# EUROPÄISCHE PATENTANMELDUNG

(11) **EP 0 823 633 A1**
(43) Veröffentlichungstag der Anmeldung: **11.02.1998**
(21) Anmeldenummer: 97112928.3
(22) Anmeldetag: 28.07.1997
(51) Int. Cl.: G01N 33/48, G01N 15/04, G01N 21/64, G01N 33/533, G01N 33/536, G01N 33/569, B04B 5/04

(54) **Verfahren zur Erfassung von Oberflächenantigenen oder Strukturmerkmalen von Zellen, Partikeln oder Makromolekülen**

(30) Priorität: 07.08.1996 DE 19631855
(71) Anmelder: Komanns, Aribert, Dr., 50129 Bergheim (DE)
(72) Erfinder: Komanns, Aribert, Dr., 50129 Bergheim (DE)

(57) **Zusammenfassung**

Werden Zellen, Partikel oder Makromoleküle mit fluoreszenzmarkierten Antikörpern oder Liganden fluoreszenzmarkiert und in lichtdurchlässigen Reaktionsgefäßen einem flüssigen oder gelartigen Medium aufgeschichtet und zentrifugiert, so kommt es zu einer Sedimentation und/oder Auftrennung der Bestandteile im Medium. Gegenüber einer Lichtquelle (6) mit fluoreszenzanregendem Spektrum entstehen Fluoreszenzbandenmuster, die visuell erfaßt oder mittels Scanner oder CCD-Sensor registriert werden. Alternativ kann während der Zentrifugation eine orts- und/oder zeitaufgelöste Fluoreszenzmessung durch Zentrifugalanalyse erfolgen. Dabei erzeugt eine stationäre oder radiär bewegliche Optik (6,7,8) einen punktförmigen monochromatischen Anregungsstrahl, der beim Auftreffen auf fluoreszenzmarkierte Bestandteile im Medium eine Fluoreszenzlichtemission erzeugt, die durch einen Fluoreszenzdetektor (7) erfaßt wird, der auf den jeweiligen Anregungsort im Medium fokusiert ist. Dabei ist gewährleistet, daß während der Zentrifugation jeweils innerhalb einer Rotorumdrehung die Fluoreszenzintensität in allen lichtdurchlässigen Reaktionsgefäßen (3) erfaßt wird. Die Steuerung übernimmt eine Zentraleinheit.

## Beschreibung

Die Erfindung betrifft ein Verfahren und eine Vorrichtung zur Erfassung von Oberflächenantigenen oder Strukturmerkmalen von Zellen, Partikeln oder Makromolekülen.

Werden Vollblut, Leukozytenkonzentrat, mononukleäre Zellen (nach Dichtegradientenzentrifugation aus Vollblut), plättchenreiches Plasma, subzelluläre Partikel oder Makromoleküle (DNA, RNA) mit fluoreszenzmarkierten Antikörpern oder Liganden inkubiert, die spezifisch gegen Oberflächenantigene oder Strukturmerkmale dieser Bestandteile gerichtet sind, so binden die fluoreszenzmarkierten Antikörper oder Liganden sich spezifisch an korrespondierende Oberflächenantigene oder Strukturmerkmale dieser Bestandteile. Dabei kann bei Bedarf eine Mehrfachmarkierung vorgenommen werden, z.B. indem man Antikörper oder Liganden unterschiedlicher Spezifität mit verschiedenen Fluorochromen koppelt, die bei Anregung Fluoreszenzlicht unterschiedlicher Wellenlängen erzeugen. Wird ein derartiger Reaktionsansatz in lichtdurchlässigen Reaktionsgefäßen einem flüssigen oder gelartigen Medium (z.B. aus Mikroglaskügelchen, Latexpartikeln, Dextran oder Zellulose) aufgeschichtet und anschließend zentrifugiert, so sedimentieren die Bestandteile im Medium gemäß ihren unterschiedlichen physikalischen Eigenschaften und gemäß der Porengröße im Gel unterschiedlich schnell, sodaß sich idealerweise eine bandenartige Auftrennung der Bestandteile abzeichnet. Die verschiedenen Banden repräsentieren z.B. unterschiedliche Zellen, die mit Ausnahme der roten Zellen keine Eigenfarbe besitzen und somit bei Tageslicht nicht sichtbar sind. Durch die spezifische Markierung von Oberflächenantigenen mit fluoreszenzmarkierten Antikörpern können alle Banden mit fluoreszenzmarkierten Bestandteilen (Zellen) gegenüber einer Lichtquelle mit fluoreszenzanregendem Spektrum sichtbar gemacht werden. Die Ablesung der Fluoreszenzbanden kann nach Abschluß der Zentrifugation visuell oder maschinell erfolgen. Dabei können die lichtdurchlässigen Reaktionsgefäße über einen Scanner mit einer beweglichen Optik, die einen punktförmigen monochromatischen Anregungsstrahl erzeugt und einen Fluoreszenzdetektor, der auf den jeweiligen Anregungsort im Reaktionsgefäß fokusiert ist, abgescannt werden. Die Fluoreszenzintensität über den Reaktionsgefäßen wird ortsauflösend erfaßt. Alternativ kann das Fluoreszenzmuster mit dem CCD-Sensor einer Kamara erfaßt werden und über ein nachgeschaltetes Computerprogramm ausgewertet werden. Eine weitere Detektionsmöglichkeit ist über eine Zentrifugalanalyse möglich. Dabei werden die Fluoreszenzintensitäten über den Reaktionsgefäßen, die in radiärer Anordnung auf einem Rotor angeordnet sind, mittels einer stationären oder radiär beweglichen Optik während der Zentrifugation orts- und/oder zeitaufgelöst registriert. Die Optik erzeugt dabei einen punktförmigen monochromatischen Anregungsstrahl, der auf das Medium innerhalb der lichtdurchlässigen Reaktionsgefäße ausgerichtet ist. Trifft der Strahl auf fluoreszenzmarkierte Bestandteile, wird eine ungerichtete Fluoreszenzstrahlung erzeugt, die durch einen Fluoreszenzdetektor erfaßt wird, der auf den jeweiligen Anregungsort im Reaktionsgefäß fokusiert ist. Bei der Zentrifugalanalyse, die ein bekanntes Rationalisierungsprinzip darstellt, können mehrere Messungen zeitgleich erfolgen und ausgewertet werden.

Aus EP 0194212 B1 ist ein Verfahren zum Nachweis einer Erythrozyten-Agglutination bekannt, bei dem eine Mischung von Serum und roten Blutkörperchen nach einer Inkubation in ein Gelmilieu gegeben wird, das antikörperhaltig oder antikörperfrei ist. Diese Mischung wird im weiterem Verlauf Sedimentationsbedingungen unterworfen, die eine Trennung von Serum und roten Blutkörperchen gewährleisten, einen Kontakt von Blutzellen und Antikörpern im Gel ermöglichen und agglutinierte Erythrozyten zurückhalten, während nicht agglutinierte Erythrozyten im Gel sedimentieren. Aufgrund der Eigenfarbe der Erythrozyten wird das unterschiedliche Sedimentationsverhalten von isolierten Erythrozyten und Agglutinaten visuell abgelesen. Die Agglutination beruht auf eine Antigen-Antikörperreaktion, die zu einer Vernetzung der beteiligten Zellen führt mit Ausbildung größerer Agglutinate, die das Gel nicht passieren können. Diese Methode findet in der Blutgruppenserologie Anwendung.

Zur Bestimmung der Antigenität von Blutzellen mittels fluoreszenzmarkierter Antikörper ist das Verfahren der Durchflußzytometrie etabliert. Hierbei durchströmen markierte Zellen hintereinander eine dünne Kapillare, die zentral von einem Laserstrahl durchleuchtet wird. Passiert eine Zelle den Laserstrahl entstehen Streulicht- und Fluoreszenzimpulse, die über Detektoren erfaßt werden. Die Streulichtimpulse ermöglichen eine Zuordnung der Zellart. Die Fluoreszenzimpulse, die durch Bindung spezifischer fluoreszenzmarkierter Antikörper entstehen, erlauben den Nachweis von Oberflächenantigenen der Zelle. Das Verfahren der Durchflußzytometrie hat sich u.a. bei der Immunphänotypisierung der Leukämien, der HLA-Typisierung vor Transplantationen, dem CD34-Monitoring im Rahmen der Blutstammzellseparation und der Bestimmung von Thrombozytenantigenen bewährt.

Das Verfahren der Zentrifugalanalyse wurde von Norman G. Anderson bereits in den sechziger Jahren in Analytical Biochemistry, Vol. 23, 207-218 (1968) unter dem Titel "Analytical Techniques for Cell Fraktions" und in Science, Vol. 166, 317-324, (1969) unter dem Titel "Computer Interfaced Fast Analyzers" beschrieben. Bei der Zentrifugalanalyse werden Untersuchungsmaterial und Reagenzien primär in getrennte Kammern eines Küvettenrotorsystems gebracht. Anschließend werden sie durch Anzentrifugieren in der Meßkammer des Küvettenrotorsystems zusammengeführt. Die Messung der Lichttransmission im Reaktionsansatz erfolgt in der Art, daß jeweils innerhalb einer Rotorumdrehung die Lichttransmissionswerte aller Meßkammern photometrisch registriert werden. Somit wird insbesondere durch die Zentrifugalanalyse ermöglicht, eine erhebliche Anzahl von gleichen Untersuchungen parallel nebeneinander her ablaufen zu lassen.

In vielen folgenden Patentanmeldungen, z.B. US 41 35883, US 3713775, DE 3044385 A1, DE 3314961 A1 wurde im Zuge der Weiterentwicklung dieses Analysenprinzips die Form der Rotoren immer komplizierter gestaltet oder die Rotortemperierung wurde weitestgehend optimiert. In US 3713775 wurde zudem der Einsatz von Vollblut im Sinne einer weiteren Automatisation vorgesehen. Dabei findet in einer integrierten Zentrifuge eine Fraktionierung der zellulären Bestandteile und des Serums statt.

In US 5 525 240 wird eine Vorrichtung und ein Verfahren offenbart, bei dem die Zentrifugation eines flüssigen Gemisches in einem Zentrifugenröhrchen in der Absicht erfolgt, Komponenten des flüssigen Gemisches mit unterschiedlicher Sedimentationsrate zu separieren. Dazu wird das Gemisch vorab mit Testpartikel versetzt, die fluoreszenzmarkiert sind und die in etwa das gleiche Sedimentationsverhalten aufweisen, wie die eigentlich interessierenden Bestandteile des Gemisches. Die Sedimentation der Testpartikel wird während der Zentrifugation verfolgt über ein Laser-Array mit korrespondierenden Photodektoren, die in fixierter radiärer Ausrichtung das Zentrifugenröhrchen mit dem Gemisch während der Zentrifugation umgeben. Auf die Weise kann eine Detektion des Sedimentationsverhaltens der Testpartikel im Röhrchen stattfinden. Die Zentrifugengeschwindigkeit und Zentrifugationsdauer wird in Abhängigkeit des Sedimentationsverhaltens der Testpartikel gesteuert, sodaß insgesamt eine optimale Separation der interessierenden Bestandteile erzielt wird.

In US 3 679 367 wird eine Vorrichtung zur Messung des Partikelanteils (Packed Volume) am Gesamtvolumen eines flüssigen Gemisches offenbart. Dabei werden z.B. Blutproben über eine spezielle axiale Einführungsvorrichtung nacheinander in ein kontinuierlich laufendes Zentrifugenrotorsystem geladen. Im Zentrifugenrotor werden die Blutproben unter der Einwirkung der Zentrifugalkraft in eine peripher lokalisierte Kapillarkammer befördert. Diese ist offenendig und hat eine serpentinenartige radiär ausgerichtete Konfiguration, d.h. die Kapillare läuft in sich zurück und bildet peripher eine Auffangschleife aus. Dadurch wird erzielt, daß ein fixes Volumen der zugeführten Blutprobe in der Schleife retiniert wird. In der Folge tritt unter der Einwirkung der Zentrifugalkraft eine Sedimentation der festen Bestandteile der Blutprobe ein. Über eine Optik mit einer Lichtquelle und einem Lichtsensor, die in Höhe der Kapillarschleife zu beiden Seiten des Zentrifugenrotors angebracht sind, wird der Sedimentationsvorgang abgescannt. Dabei ist in den Strahlengang ein über einen Motor drehbarer Spiegel angebracht, der eine Ablenkung des Lichtstrahles über die volle Länge der Kapillarschleife ermöglicht. Eine nachgeschaltete Elektronik gewährleistet die Auswertung der Sedimentationsergebnisse und die Errechnung des "Packed volume".

Aus DE 4041554 C2 ist ein Verfahren zur Zentrifugalanalyse bekannt, bei dem die Sedimentationskinetik von Blutzellen, Hämagglutinaten und AntigenAntikörperkomplexen unter Einwirkung von Zentrifugalkräften registriert wird. Dabei wird speziell eine lichtoptische Messung durch mehrere stufenweise radiär angeordnete Photometer, Reflektometer bzw. Zeilendioden oder über ein radiär bewegliches Photometer bzw. Reflektometer über der gesamten Meßkammer durchgeführt.
Beim Verfahren nach EP 0194212 B1 findet eine Bestimmung von Oberflächenantigen oder Strukturmerkmalen von Erythrozyten durch eine Agglutinationsreaktion unter Verwendung nichtmarkierter spezifischer Antikörper statt. Nur aufgrund der Eigenfarbe der Erythrozyten kann eine Ablesung des Sedimentationsverhaltens dieser Zellen erfolgen. Bestandteile ohne Eigenfarbe sind einer Untersuchung hierbei nicht zugänglich. Als Standardverfahren zur Erfassung von Oberflächenantigenen oder Strukturmerkmalen von Zellen, Partikeln oder Makromolekülen unter Verwendung fluoreszenzmarkierter Antikörper oder Liganden gilt die oben beschriebene Durchflußzytometrie. Trotz ihrer gut reproduzierbaren Ergebnisse ist die Durchflußzytometrie als ein technisch aufwendiges und kosten- und zeitintensives Verfahren einzustufen, welches immer nur die gleichzeitige Durchführung einer einzigen Analyse erlaubt. Bei einer größeren Analysenzahl werden rasch die Grenzen dieser Methode erreicht. Alle aufgeführten Verfahren zur Zentrifugalanalyse sind zur Erfassung von Oberflächenantigenen oder Strukturmerkmalen von Zellen, Partikeln oder Makromolekülen unter Verwendung spezifischer fluoreszenzmarkierter Antikörper oder Liganden nicht geeignet. In DE 4041554 C2 erfolgt zwar eine ortsaufgelöste Transmissions- bzw. Reflexionsmessung in den Meßkammern von Küvetten, jedoch ist keine Aufschichtung der Bestandteile über ein flüssiges oder gelartiges Medium vorgesehen, sodaß auch keine bandenartige Auftrennung der Bestandteile erfolgt. Darüberhinaus ist in keinem der aufgeführten Verfahren zur Zentrifugalanalyse eine ortsaufgelöste Messung der Fluoreszenzintensität vorgesehen. Letzteres setzt eine radiär bewegliche Optik voraus, die eine punktförmige monochromatische Anregungsstrahlung erzeugt in einem fluoreszenzanregendem spektralem Bereich und einen Fluoreszenzdetektor beinhaltet, der auf den jeweiligen Anregungsort im Medium fokusiert ist und der über einen Emissionsmonochromator verfügt, der nur im spektralen Bereich des erzeugten Fluoreszenzlicht durchlässig ist, und die Wellenlänge des Anregungsstrahls unterdrückt.

Der Erfindung liegt die Aufgabe zugrunde, ein Verfahren und eine Vorrichtung anzugeben, welches zur Erfassung von Oberflächenantigenen oder Strukturmerkmalen von Zellen, Partikeln oder Makromolekülen unter Verwendung spezifischer fluoreszenzmarkierter Antikörper oder Liganden geeignet ist und darüberhinaus technisch einfach realisiert werden kann und einen hohen Probendurchsatz ermöglicht.

Die Aufgabe wird erfindungsgemäß dadurch gelöst, daß eine Fluoreszenzmarkierung von Oberflächenantigenen oder Strukturmerkmalen von Zellen, Partikeln oder Makromolekülen stattfindet, und zwar durch Inkubation mit Reagenzien, die fluoreszenzmarkierte Antikörper oder Liganden beinhalten. Die fluoreszenzmarkierten Bestandteile werden anschließend einem flüssigen oder gelartigen Medium aufgeschichtet, das sich in lichtdurchlässigen Reaktionsgefäßen befindet. Die lichtdurchlässigen Reaktionsgefäße werden z.B. in einer radiären Anordnung auf einem Rotor zentrifugiert, sodaß eine Sedimentation und/oder Auftrennung der fluoreszenzmarkierten Bestandteile im Medium resultiert. Alternativ können auch nichtmarkierte Zellen, Partikel oder Makromoleküle in lichtdurchlässigen Reaktionsgefäßen auf ein flüssiges oder gelartiges Medium aufgeschichtet werden, in das fluoreszenzmarkierte Antikörper oder Liganden gelöst sind. Bei Zentrifugation der lichtdurchlässigen Reaktionsgefäße z.B. in einer radiären Anordnung auf einem Rotor tritt eine Sedimentation und/oder Auftrennung der Bestandteile ein. Schon während der Zentrifugation treten die Bestandteile im Medium in Kontakt mit fluoreszenzmarkierten Antikörpern oder Liganden, sodaß eine Fluoreszenzmarkierung stattfindet. Nach der Zentrifugation findet durch eine Lichtquelle mit einem fluoreszenzanregenden spektralen Bereich eine Fluoreszenzanregung der fluoreszenzmarkierten Bestandteile in den lichtdurchlässigen Reaktionsgefäßen statt. Dabei tritt eine ortsaufgelöste Änderung der Fluoreszenzintensität im Medium auf, z.B. mit spezifischen Bandenmustern, die visuell ausgewertet werden können oder über einen Scanner mit einer beweglichen Optik, die einen punktförmigen monochromatischen Anregungsstrahl erzeugt und einen Fluoreszenzdetektor beinhaltet, der auf den jeweiligen Anregungsort im Medium fokusiert ist, abgescannt werden. Alternativ kann das Fluoreszenzmuster mit dem CCD-Sensor einer Kamara erfaßt werden und nach der Digitalisierung über ein nachgeschaltetes Computerprogramm ausgewertet werden. Neben einer Auswertung der ortsaufgelösten Fluoreszenzintensität nach Abschluß der Zentrifugation ist auch eine Auswertung während der Zentrifugation über eine Zentrifugalanalyse möglich. Dabei werden die Fluoreszenzintensitäten über den Reaktionsgefäßen, die in radiärer Anordnung auf einem Rotor angeordnet sind, mittels einer stationären oder radiär beweglichen Optik während der Zentrifugation orts- und/oder zeitaufgelöst registriert. Die Optik erzeugt dabei einen punktförmigen monochromatischen Anregungsstrahl, der auf das Medium innerhalb der lichtdurchlässigen Reaktionsgefäße ausgerichtet ist. Trifft der Strahl auf fluoreszenzmarkierte Bestandteile, wird eine ungerichtete Fluoreszenzstrahlung erzeugt, die durch einen Fluoreszenzdetektor erfaßt wird, der auf den jeweiligen Anregungsort im Reaktionsgefäß fokusiert ist. Dabei ist gewährleistet, daß während der Zentrifugation jeweils innerhalb einer Rotorumdrehung die Fluoreszenzintensitätswerte in allen lichtdurchlässigen Reaktionsgefäßen erfaßt werden und daß bei radiärer Bewegung der Optik eine orts- und zeitaufgelöste Messung der Fluoreszenzintensitäten über den Reaktionsgefäßen erfolgt. Die Steuerung des Rotors, der radiären Bewegung der Optik und die Meßwertaufnahme erfolgt über eine Schnittstelle durch eine Zentraleinheit. Die Zentraleinheit gewährleistet weiter die Verarbeitung der Meßwerte.

Die mit der Erfindung erzielten Vorteile gegenüber dem bisherigen Standardverfahren der Durchflußzytometrie sind in der einfachen Technik begründet, die einen hohen Probensatz bei geringem Kostenaufwand ermöglicht.

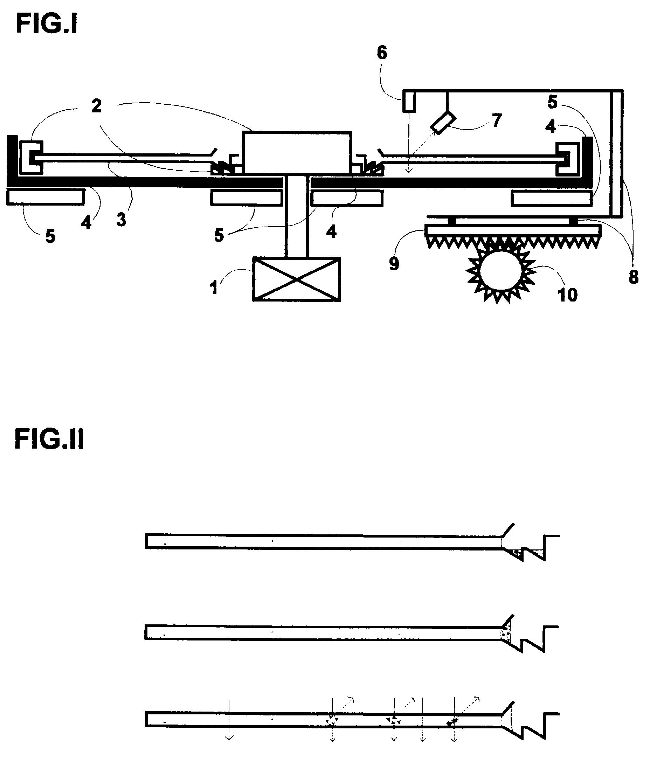

Ein Ausführungsbeispiel der Erfindung ist in der Zeichnung dargestellt und wird im folgenden näher beschrieben.

Fig.I zeigt einen Querschnitt durch eine Vorrichtung zur automatischen Auswertung des Verfahrens, wobei die wesentlichen Bestandteile herausgestellt werden. Auf die Darstellung bekannter Automatisationsmechanismen wurde bewußt verzichtet.

Fig.II zeigt drei verschiedene Phasen des verfahrensgemäßen Reaktionsablaufes in drei lichtdurchlässigen Reaktionsgefäßen in vergrößerter Darstellung.

In Fig.I ist ein Rotor (2) befestigt auf der Achse eines Motors (1) dargestellt. Der Rotor (2) ist in einer zylinderförmigen Einfassung (4) positioniert, die peltierelementgesteuert (5) beheizt wird. In den Rotor (2) sind radiäre Aussparungen eingelassen zur Aufnahme von lichtdurchlässigen Reaktionsgefäßen (3). Eine Optik, bestehend aus einer monochromatischen Lichtquelle (6), einem Fluoreszenzdetektor (7) und einer Halterung (8) ist beweglich gelagert und radiär über eine Zahnstange (9) und einen Schrittmotor (10) über den Rotorradius verschiebbar.

In Fig.II sind drei lichtdurchlässige Reaktionsgefäße in einer vergrößerten Darstellung übereinander angeordnet. Die lichtdurchlässigen Reaktionsgefäße sind einseitig verschlossene Kapillaren, die an der offenen Seite konisch aufgeweitet sind und hier über getrennte Kammern zur Aufnahme von Probe und Reagenz verfügen. Die Kapillaren sind bis auf die konische Aufweitung mit einem Medium vorgefüllt. Der obere Meniskus des Mediums ist am inneren Konusrand angedeutet. Im oberen Reaktionsgefäß sind in den getrennten Kammern eine Zellsuspension und ein Reagenz mit fluoreszenzmarkierten Antikörpern vorgelegt. Dies ist der Ausgangszustand vor Zentrifugation. Im mittleren Reaktionsgefäß ist die Situation zu Beginn der Zentrifugation dargestellt. Jetzt werden die Zellsuspension und das Reagenz in die konische Aufweitung geschleudert und dem Medium aufgeschichtet. Hierbei vermischt sich die Zellsuspension mit dem Reagenz und es findet eine Fluoreszenzmarkierung der Zellen statt. Im unteren Reaktionsgefäß ist die Sedimentation der fluoreszenzmarkierten Zellen im Medium nach längerer Zentrifugation dargestellt. Dabei sind unterschiedliche Zellarten im Medium unterschiedlich weit sedimentiert, sodaß zellspezifische Banden entstehen. Trifft während der Zentrifugalanalyse bei der radiären Bewegung der Optik ein monochromatischer Lichtstrahl eines fluoreszenzanregenden spektralen Bereiches auf eine fluoreszenzmarkierte Zelle, so entsteht Fluoreszenz- und Streulicht. Der Strahlengang ist angedeutet.

## Patentansprüche

1. Verfahren zur Erfassung von Oberflächenantigenen oder Strukturmerkmalen von Zellen, Partikeln oder Makromolekülen, bei dem die zu untersuchenden Bestandteile mittels fluoreszenzmarkierter Antikörper oder Liganden einfach- oder mehrfachfluoreszenzmarkiert werden,
dadurch gekennzeichnet, daß in lichtdurchlässigen Reaktionsgefäßen fluoreszenzmarkierte Bestandteile einem flüssigen oder gelartigen Medium aufgeschichtet werden oder nichtmarkierte Bestandteile einem flüssigen oder gelartigem Medium mit fluoreszenzmarkierten Antikörpern oder Liganden aufgeschichtet werden, daß eine Zentrifugation der Reaktionsgefäße auf einem Rotor die Sedimentation und/oder Auftrennung der Bestandteile im Medium verursacht und bei primärer Aufschichtung nicht markierter Bestandteile auf ein flüssiges oder gelartiges Medium mit fluoreszenzmarkierten Antikörpern oder Liganden eine Bindung zwischen den nichtmarkierten Bestandteilen an die fluoreszenzmarkierten Antikörper oder Liganden eintritt, daß die Sedimentation und/oder Auftrennung der fluoreszenzmarkierten Bestandteile im Medium bei Anregung mit Licht eines fluoreszenzanregenden spektralen Bereiches mit einer orts- und/oder zeitaufgelösten Änderung der Fluoreszenzintensität im Medium einhergeht, die entweder nach Abschluß der Zentrifugation gegenüber einer Lichtquelle mit fluoreszenzanregendem Spektrum visuell abgelesen oder über Scanner oder Kamara maschinell erfaßt wird oder deren Registrierung während der Zentrifugation durch Zentrifugalanalyse erfolgt.

2. Verfahren nach Anspruch 1,
dadurch gekennzeichnet, daß bei der Zentrifugalanalyse neben einem Rotor zur Aufnahme lichtdurchlässiger Reaktionsgefäße eine stationäre oder radiär bewegliche Optik eingesetzt wird, die eine punktförmige monochromatische Anregungsstrahlung in einem fluoreszenzanregenden spektralen Bereich erzeugt und die Fluoreszenzdetektoren beinhaltet, die auf den jeweiligen Anregungsort in den lichtdurchlässigen Reaktionsgefäßen fokusiert sind und nur im spektralen Bereich des Fluoreszenzlichtes empfindlich sind und daß die Optik während der Zentrifugation jeweils innerhalb einer Rotorumdrehung die Fluoreszenzintensitätswerte in allen lichtdurchlässigen Reaktionsgefäßen erfaßt und bei radiärer Bewegung eine orts- und zeitaufgelöste Messung der Fluoreszenzintensitäten über der gesamten Länge der lichtdurchlässigen Reaktionsgefäße ermöglicht, oder daß bei einer stationären Optik ein radiär beweglicher Rotor realisiert wird.

3. Verfahren nach Anspruch 1,
dadurch gekennzeichnet, daß orts- und zeitgleich zur Messung der Fluoreszenzintensität im Reaktionsgefäß zusätzlich eine Messung der Lichttransmission oder der Lichtreflexion erfolgt.

4. Verfahren nach Anspruch 1,
dadurch gekennzeichnet, daß die zu untersuchenden Bestandteile unabhängig von der notwendigen Fluoreszenzmarkierung vor der Aufschichtung auf das Medium eine Reaktion mit weiteren Substraten eingehen oder während der Zentrifugation eine Reaktion mit weiteren im Medium gelösten Substraten eingehen, die entweder lysierende Wirkung entfalten, das Sedimentationsverhalten der Bestandteile verändern, eine funktionsaktivierende Wirkung auslösen oder die Bindung der fluoreszenzmarkierten Antikörper oder Liganden beeinflussen.

5. Vorrichtung zur Durchführung des Verfahrens nach Anspruch 1,2,3 oder 4 mit einem motor- oder schrittmotorgetriebenen (1) Rotor (2), in den radiär angeordnete Aussparungen eingelassen sind, die zur Aufnahme von lichtdurchlässigen Reaktionsgefäßen (3) dienen und mit einer Optik (6,7,8) zur Messung der Fluoreszenzintensität,
dadurch gekennzeichnet, daß die Optik (6,7,8) stationär oder radiär beweglich angeordnet ist und eine Lichtquelle (6) mit einem monochromatischen punktförmigen Anregungsstrahl und einem auf den Anregungsort im Reaktionsgefäß (3) fokusierten Fluoreszenzdetektor (7) beinhaltet.

6. Vorrichtung zur Durchführung des Verfahrens nach Anspruch 1,2,3 oder 4 mit einem motor- oder schrittmotorgetriebenen (1) Rotor (2), in den radiär angeordnete Aussparungen mit vertikalen Lichtschlitzen eingelassen sind, die zur Aufnahme von lichtdurchlässigen Reaktionsgefäßen (3) dienen und mit einer Optik (6,7,8) zur kombinierten Messung der Fluoreszenzintensität sowie der Lichttransmission oder Lichtreflektion,
dadurch gekennzeichnet, daß die Optik (6,7,8) stationär oder radiär beweglich angeordnet ist und eine Lichtquelle (6) mit einem monochromatischen punktförmigen Anregungsstrahl und Fluoreszenz- sowie Lichttransmissions- oder Lichtreflektionsdetektoren (7) beinhaltet, die auf den Anregungsort im Reaktionsgefäß (3) fokusiert sind.

7. Vorrichtung nach Anspruch 5 oder 6,
dadurch gekennzeichnet, daß der Rotor (2) in einer zylinderförmigen Einfassung (4) positioniert ist, deren Material eine hohe Wärmeleitfähigkeit besitzt und deren Temperatur peltierelementgesteuert (5) eingestellt wird.

8. Vorrichtung nach Anspruch 5,
dadurch gekennzeichnet, daß die Optik (6,7,8) aus einer monochromatischen Lichtquelle (6), z.B. in Form eines Lasers, aus einem Fluoreszenzdetektor (7) mit Emissionsmonochromator, Photomultiplier oder Fotodiode bzw. Fototransistor sowie aus einer Halterung (8) besteht.

9. Vorrichtung nach Anspruch 5 oder 6,
dadurch gekennzeichnet, daß die Optik (6,7,8) auf einer Zahnstange (9) befestigt schrittmotorgetrieben (10) radiär zur Ausrichtung des Rotors (2) bewegt werden kann.

10. Vorrichtung nach Anspruch 5 oder 6,
dadurch gekennzeichnet, daß die Reaktionsgefäße (3) aus einseitig verschlossenen Kapillaren bestehen, die an der offenen Seite konisch aufgeweitet sind und hier optional getrennte Kammern zur Aufnahme von Probe und Reagenz integriert werden können, die vor Zentrifugationsbeginn eine Trennung von Reaktionspartnern gewahrleisten.

11. Vorrichtung nach Anspruch 5,6 oder 10,
dadurch gekennzeichnet, daß die mit flüssigem oder gelartigem Medium vorgefüllten Reaktionsgefäße (3) durch eine dünne Folie luftdicht verschlossen sind.

12. Vorrichtung nach Anspruch 5,6,10 oder 11,
dadurch gekennzeichnet, daß mehrere Reaktionsgefäße (3) zu einem kompakten Kranz aus Kunststoff als Einmalartikel zusammengefaßt sind.

13. Vorrichtung nach Anspruch 5 oder 6,
dadurch gekennzeichnet, daß ein Pipettiermechanismus zur Vorgabe von Probe und Reagenz vorgesehen ist.

14. Vorrichtung nach Anspruch 5,6 oder 13,
dadurch gekennzeichnet, daß eine integrierte Waschstation zur Reinigung des Pipettiersystems existiert.

15. Vorrichtung nach Anspruch 5,6 oder 13,
dadurch gekennzeichnet, daß in das Pipettiersystem ein Mechanismus zur automatischen Aufnahme bzw. zum Abwurf von Pipettenspitzen integriert ist für einen Pipettenwechsel zwischen den Pipettierschritten.

16. Vorrichtung nach Anspruch 5,6,13,14 oder 15,
dadurch gekennzeichnet, daß der Dosierer des Pipettiersystems über einen Dilutor verfügt, der Volumina bis zu 2 µl ausreichend genau zu dosieren vermag.

17. Vorrichtung nach Anspruch 5 oder 6,
dadurch gekennzeichnet, daß in die Vorrichtung beheizte Inkubatorblöcke zur Aufnahme von Untersuchungsmaterial und Reagenzien integriert sind.
